# EUROPÄISCHE PATENTANMELDUNG

(11) **EP 2 698 136 A1**
(43) Veröffentlichungstag der Anmeldung: **19.02.2014**
(21) Anmeldenummer: 12186197.5
(22) Anmeldetag: 26.09.2012
(51) Int. Cl.: A61F 13/02, A61H 39/04

(54) **Kosmetikpflaster**

(30) Priorität: 17.08.2012 DE 102012107584
(71) Anmelder: Teigelkämper, Gordon, 58313 Herdecke (DE); Malisch, Christoph, 85540 Haar (DE)
(72) Erfinder: Teigelkämper, Gordon, 58313 Herdecke (DE); Malisch, Christoph, 85540 Haar (DE)
(74) Vertreter: Kisters, Michael Marcus

(57) **Zusammenfassung**

Die Erfindung betrifft ein Pflaster zur Aufbringung auf die Haut für kosmetische Zwecke, mit einer Klebeschicht und optional einer Deck- und Matrixschicht wobei auf die Klebeschicht ein oder mehrere Druckkörper mit einem mittleren Durchmesser von 0,5 bis 5 mm angeordnet sind.

Weiterhin betrifft die Erfindung ein Verfahren zur kosmetischen Akupressur der menschlichen Haut durch Aufkleben des Pflasters auf die Haut.

## Beschreibung

Pflaster sind neben der reinen Wundabdeckung in vielen Ausführungen zu therapeutischen Anwendungen in der Medizin und der Kosmetik bekannt. Beispielhaft für die große therapeutische Verwendungsbreite sei DE102005053909A1 genannt, wo eine Hautauflage mit einer auf der Haut haftenden Matrix, enthaltend mindestens einen Wirkstoff zur Behandlung von Cellulite, beschrieben ist.

In kosmetischen Anwendungen von Pflastern kommen als kosmetisch wirkender Stoff häufig Metalle oder Edelmetalle zum Einsatz. DE 60028642 T2 beschreibt z.B. die Einarbeitung von magnetischen Partikeln auf Ferritbasis mit Zink und Mangan in eine Matrix, um ein Pflaster mit magnetischer Wirkung zu erhalten.

In DE102008011566A1 ist ein Pflaster mit einer metallisierten Polyester-Folie offenbart, die sehr dünn ist und nur geringen Druck auf die Haut ausübt.

DE102008035848A1 betrifft ein Pflaster mit einer Folie, die auf beiden Seiten mit einer therapeutisch wirksamen Beschichtung wie z.B. einer Goldfolie versehen ist.

Die bekannten Pflaster sind aber nur bedingt zur kosmetischen Behandlung von Hautfalten geeignet.

Es zeigte sich, dass die Ausübung von geringem punktuellem Druck auf Akupressurpunkte z.B. eine Straffung der Haut bewirkt und die Faltenbildung reduziert.

Gegenstand der vorliegenden Erfindung sind daher Pflaster zur Aufbringung auf die Haut für kosmetische Zwecke mit einer Klebeschicht wobei auf der Klebeschicht ein oder mehrere Druckkörper mit einem mittleren Durchmesser von 0,5 bis 5 mm angeordnet sind. Im Verfahren nach der Erfindung wird das Pflaster wird mit der Klebeschicht auf der Haut fixiert. Hier durch übt der Druckkörper einen mechanischen Druck auf das darunter liegende Gewebe aus.

Die Klebeschicht der erfindungsgemäßen Pflaster können eine nicht klebende Deckschicht aufweisen. Es ist auch möglich, eine nicht klebende Deckschicht mit einer Klebeschicht zu versehen.

Optional ist zwischen Deckschicht und Klebeschicht der Pflaster eine Matrixschicht angeordnet, wobei die Matrixschicht eine oder mehrere Druckkörper aufweist.

Die Deckschicht kann beispielsweise aus beschichtetem Papier, Polysilikon, Polyethylen, Polypropylene, Copolymere von Ethylen und Vinylacetat, Polyester, Polyurethanen, Polyamid, Polyacrylat und Polyisobutylen bestehen. Die Deckschicht kann eine an sich beliebige Dicke besitzen, aus Gründen des Tragekomforts bieten sich Dicken von ca. 50 pm bis etwa 1,0 mm an. Die Deckschicht kann in Form einer geschlossenen oder perforierten Folie, einem Gewebe oder geschlossenen oder perforierten Nonwoven eingesetzt werden. Um den Druckkörper besser platzieren zu können, ist es vorteilhaft, die Deckschicht und optional die Matrixschicht ganz oder zumindest im Bereich der Druckkörper transparent zu gestalten.

Deckschicht und Klebeschicht können mit einem geeigneten Kleber miteinander verklebt werden. Vorzugsweise werde mit dem gleichen Kleber die Druckkörper zwischen Deck- und Matrixschicht fixiert. Alternativ können die Druckkörper auf oder in die Deckschicht und/oder Klebeschicht durch Erwärmen, d.h. ohne Klebemittel, fixiert werden.

Die Matrixschicht kann ebenfalls zur Aufnahme und Fixierung der Druckkörper dienen und kann im Prinzip aus den gleichen Materialien wie die Deckschicht bestehen. Es ist möglich, die Druckkörper auf eine Matrixschicht mit einem geeigneten Kleber zu fixieren und dann vorzugsweise mit dem gleichen Kleber Deck- und Matrixschicht miteinander zu verbinden. Alternativ können die Druckkörper auf oder in die Matrixschicht durch Erwärmen, d.h. ohne Klebemittel, eingebracht werden. Eine so hergestellte Matrixschicht kann wiederum durch Verkleben oder Erwärmen mit der Deckschicht verbunden werden. In einer weiteren Ausführungsform können die Druckkörper in eine Matrixschicht eingebettet werden, ohne diese zu durchdringen. In diesem Fall sind Deck- und Matrixschicht identisch, und die außen liegende Schicht wird als Deckschicht bezeichnet.

Die Klebeschicht kann aus jedem hautverträglichen Klebemittel bestehen, das für die dermatologische Verwendung zugelassen ist. Dem Fachmann sind solche Klebesysteme, z.B. auf Basis von in Wasser gelbildeten Polymeren, Polyisobutylen, Cataplasmen, Polyacrylsäure oder Polyacrylaten bekannt. Die Klebeschicht ist bevorzugt wasserresistent, um ein Verrutschen des Pflasters beim Schwitzen zu vermeiden.

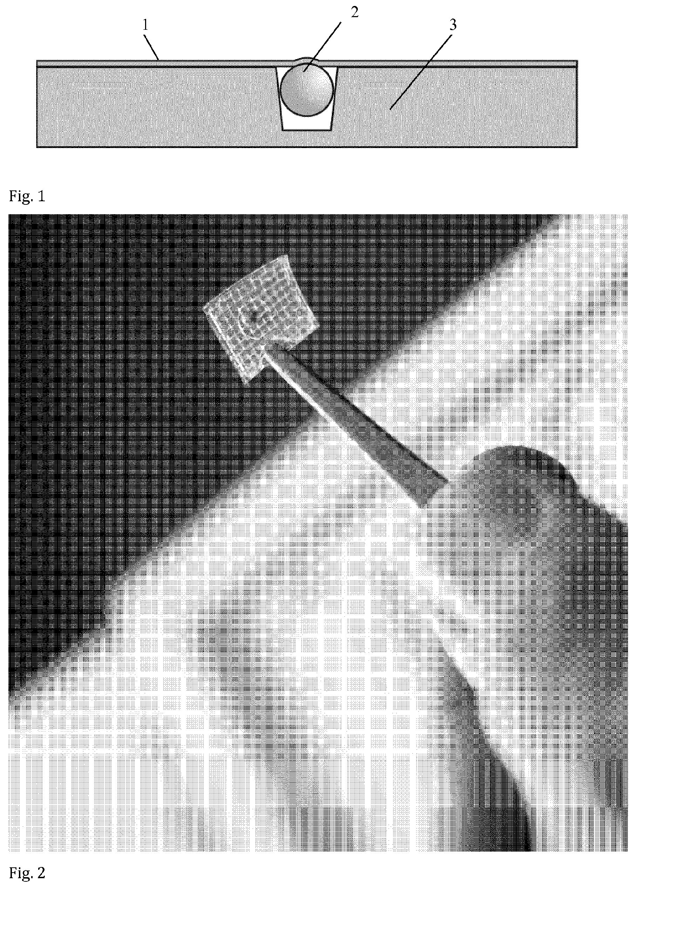

Fig. 1 zeigt ein erfindungsgemäßes Pflaster in schematischer Seitenansicht, wobei (1) für die Klebeschicht, (2) für den Druckkörper und (3) für die Matrixschicht, hier kombiniert mit der Deckschicht, steht.

In einer besonderen Ausführungsform der Erfindung besitzt die Klebeschicht an mindestens einer Stelle eine Aussparung bzw. ist nicht klebend ausgeführt. Dies dient zu einer leichten Handhabung beim Auflegen auf die Haut bzw. bei der Entnahme aus dem Vorratsbehälter, jeweils z.B. mit einer Pinzette. Fig. 2 zeigt ein Pflaster mit einer solchen Ausnehmung für eine Pinzette.

Die erfindungsgemäß verwendeten Druckkörper können im Prinzip jede geometrische Form besitzen, wie z.B. die Form einer Kugel, Ellipsoid, Kegel, Pyramide oder Kubus. Die weitgehend kugelförmige Form der Druckkörper ist bevorzugt. Die Oberfläche der Druckkörper kann rauh, glatt uneben oder texturiert sein. Der Durchmesser des Druckkörpers ist auf die größte Ausdehnung in einer Raumachse bezogen.

Als Material für die Druckkörper kommen alle im pH-Bereich der menschlichen Haut nicht korrodierenden oder oxidierenden sowie nicht-toxische oder nicht-allergene Materialien in Frage, wie z.B. nicht-ferromagnetische Metalle wie Chrom, Titan, Gold, Silber, Palladium oder Platin. Ebenso können Druckkörper aus Keramik, Polymeren (wie z.B die für die Deckschicht genannten Materialien), Hölzer wie Eiche oder Esche oder Pflanzensamen wie Semen Vaccariae (Wang Bu Liu Xing) verwendet werden. Bevorzugt enthalten die erfindungsgemäßen Pflaster einen einzelnen Druckkörper, der in der Mitte des Pflasters angeordnet ist. Der mittlere Durchmesser der Druckkörper beträgt bevorzugt 1 bis 2 mm.

Fig. 2 zeigt ein erfindungsgemäßes Pflaster vor der Anwendung.

Erfindungsgemäße Pflaster können eine Größe von 0,5 bis 4 cm² besitzen und rechteckig, quadratisch, rund, elliptisch oder trapezoid sein. Bevorzugt sind die Pflaster quadratisch mit ca. 6 mm Kantenlänge oder rechteckig mit 5 - 10 mm Kantenlänge.

Die Deckschicht, die Klebeschicht und/oder die Matrixschicht können aromatische Öle oder Parfüms enthalten. Weiterhin können diese Schichten zur Verbesserung des Tragekomforts durchlässig für Luft und Feuchtigkeit sein.

Weiterhin ist Gegenstand der vorliegenden Erfindung ein Verfahren zur kosmetischen Akupressur, insbesondere zur kosmetischen Behandlung von Hautfalten, zum Lifting von atrophierter Haut, zur kosmetischen Behandlung von altersbedingter Fleckenbildung auf der Haut und/oder zur kosmetischen Behandlung von sichtbaren Äderchen durch Aufkleben eines erfindungsgemäßen Pflasters auf die menschliche Haut für 1 bis 12 Stunden.

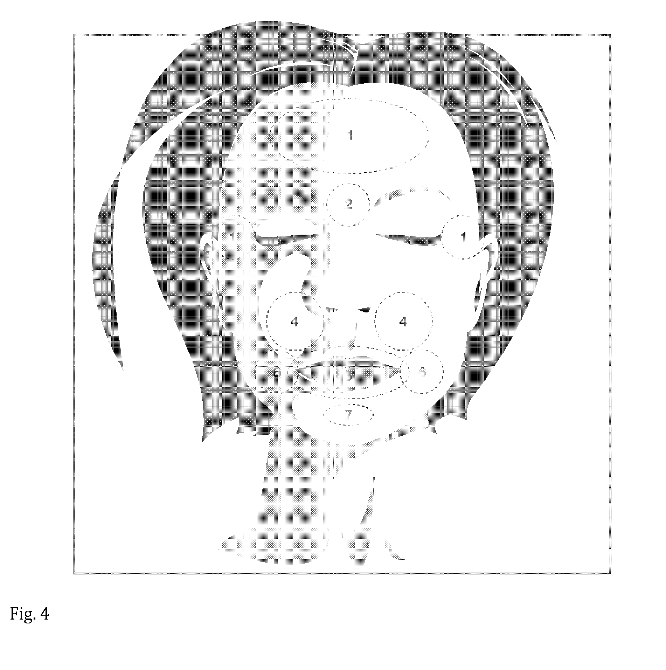

Das Pflaster wird dabei bevorzugt auf Akupressurpunkte der Haut aufgeklebt. Die Akupressurpunkte sind dem Fachmann bekannt und werden auch als Akupunkturpunkte bezeichnet. Für die erfindungsgemäße kosmetische Behandlung geeignete Akupressurpunkte sind in den Fig. 4 - 8 gezeigt.

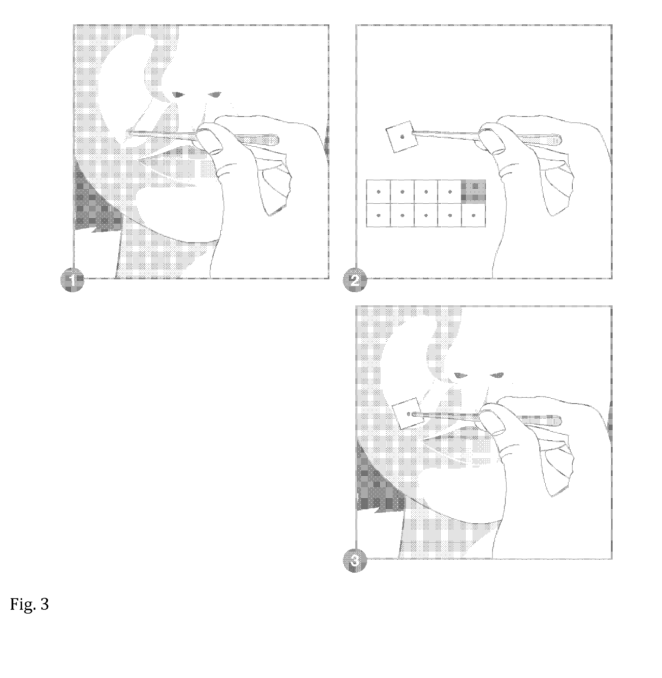

Am wirkungsvollsten ist die kosmetische Akupressur nach dem erfindungsgemäßen Verfahren, wenn sie abends, vor dem Schlafengehen, angewendet wird. Das Verfahren ist schematisch in Fig. 3 gezeigt. Nach einer gründlichen Gesichtsreinigung wird bevorzugt mit einer Pinzette ein leichter Druck auf den für die jeweilige kosmetische Behandlung geeigneten Akupressurpunkt ausgeübt, bis eine kleine Mulde entsteht. Schmerzt der Druckpunkt leicht, wurde die richtige Stelle gefunden. Nun wird mit einer Pinzette ein erfindungsgemäßes Pflaster auf die Delle des Druckpunktes aufgebracht und dieses kreisend ganz leicht, pro Punkt ca. 10 Sekunden lang, einmassiert. Bevorzugt wird das Verfahren für 1 - 6 Wochen zweimal pro Woche durchgeführt.

Erfindungsgemäße Pflaster werden bevorzugt auf die Haut im Gesichts- und Halsbereich, insbesondere wie in Fig. 4 gezeigt, an der Stirn, seitlich und unterhalb der Augen sowie seitlich von Nase und Mund, an den Augenlidern, Wangen oder Arealen seitlich der Mundwinkel sowie des gesamten Halsbereichs aufgebracht.

Besonders geeignete Regionen oder Akupressurpunkte zur Behandlung mit dem erfindungsgemäßen Pflaster sind in den Fig. 4 bis 8 dargestellt und im Folgenden beschrieben.

Fig. 4 zeigt die Lokalisation der folgenden Faltenregionen
1. GALLENBLASENFALTEN
   Lange, wellenartig horizontal verlaufende Falten an der Stirn sowie radiär verlaufende Falten an den äußeren Augenwinkeln
2. LEBERFALTEN
   Kurze, vertikal verlaufende Falten am inneren Ende der Augenbrauen
4. LUNGENFALTEN
   Lange, vertikal verlaufende Falten seitlich der Nasenflügel
5. MILZFALTEN
   Radiär verlaufende Falten um den Mund herum
6. DICKDARMFALTEN
   Vertikal verlaufende Falten seitlich der Mundwinkel
7. REN-MAI-FALTEN
   Kurze, vertikal verlaufende Falten unterhalb der Unterlippe

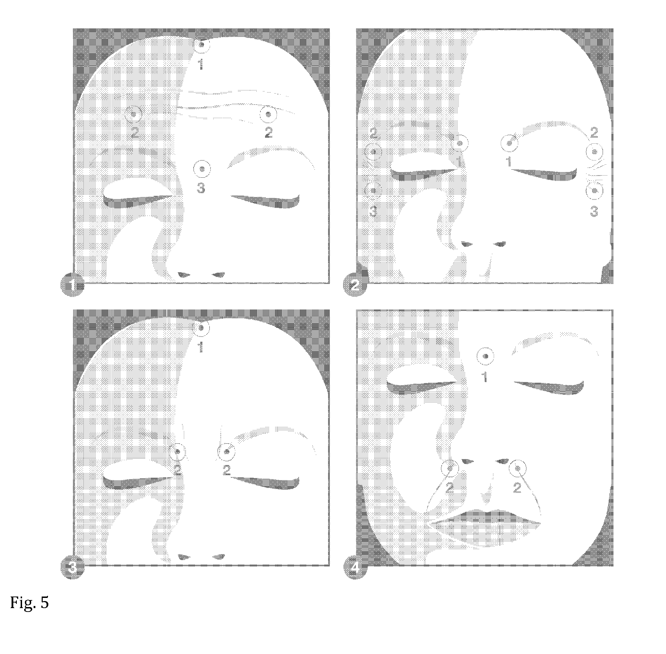

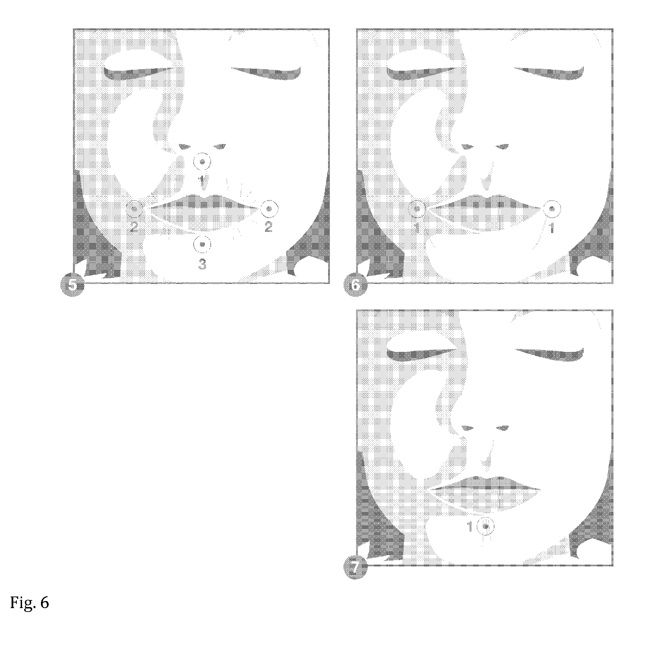

Fig. 5 und 6 zeigen die Lage der Akupressurpunkte in den Faltenregionen
Abb. 1: Gallenblasenfalten an der Stirn (lange, wellenartig, horizontal verlaufende Falten an der Stirn)
   1. Extra Punkt 1: Auf der Kopfmittellinie direkt an der Haargrenze
   2. Gallenblase 14: Eine eigene Daumenbreite oberhalb der Mitte der Augenbrauen
   3. Yin Tang: Auf der Kopfmittellinie zwischen den Augenbrauen an der tiefsten Stelle
Abb. 2: Gallenblasenfalten seitlich und unterhalb der Augen (Radiär verlaufende Falten an den äußeren Augenwinkeln)
   1. Blase 2: Am inneren Ende der Augenbrauen
   2. Dreifacher Erwärmer 23: In der Vertiefung am äußeren Ende der Augenbrauen
   3. Extra Punkt 2: Zwei Daumenbreiten senkrecht unterhalb des Punktes Nr.2
Abb. 3: Leberfalten (kurze, vertikal verlaufende Falten am inneren Ende der Augenbrauen)
   1. Extra Punkt 1: Auf der Kopfmittellinie direkt an der Haargrenze
   2. Blase 2: Am inneren Ende der Augenbrauen
Abb. 4: Lungenfalten (lange, vertikal verlaufende Falten seitlich der Nasenflügel)
   1. Yin Tang: Auf der Kopfmittellinie zwischen den Augenbrauen an der tiefsten Stelle
   2. Dickdarm 20: Seitlich der Nasenflügel
Abb. 5: Milzfalten (radiär verlaufende Falten um den Mund herum)
   1. Ren Mai 26: Auf der Kopfmittellinie direkt unterhalb der Nase
   2. Magen 4: 0,5 eigene Daumenbreiten seitlich der Mundwinkel
   3. Du Mai 24: In einer Vertiefung unterhalb der Lippe und in der Mitte des Unterkiefers
Abb. 6: Dickdarmfalten (vertikal verlaufende Falten seitlich der Mundwinkel)
   1. Magen 4: 0,5 eigene Daumenbreiten seitlich der Mundwinkel
Abb.7: Ren-Mai-Falten (kurze, vertikal verlaufende Falten unterhalb der Unterlippe)
   1. Ren Mai 24: In einer Vertiefung unterhalb der Lippe und in der Mitte des Unterkiefers

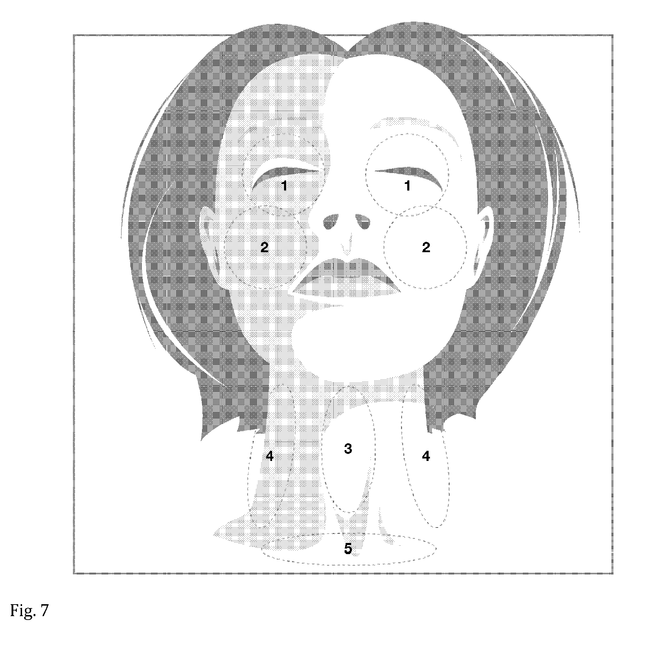

Fig. 7 zeigt die Lokalisation der folgenden Atrophie-Regionen
1. MAGEN-REGION
   Atrophie und Erschlaffung der Haut der oberen und unteren Augenlider
2. DÜNNDARM-REGION
   Atrophie und Erschlaffung der Haut im Wangenbereich
3. REN MAI-REGION
   Atrophie und Erschlaffung der Haut im vorderen Halsbereich
4. MAGEN DARM-REGION
   Atrophie und Erschlaffung der Haut im seitlichen Halsbereich
5. UNTERE MAGEN-REGION
   Atrophie und Erschlaffung der Haut im unteren Halsbereich

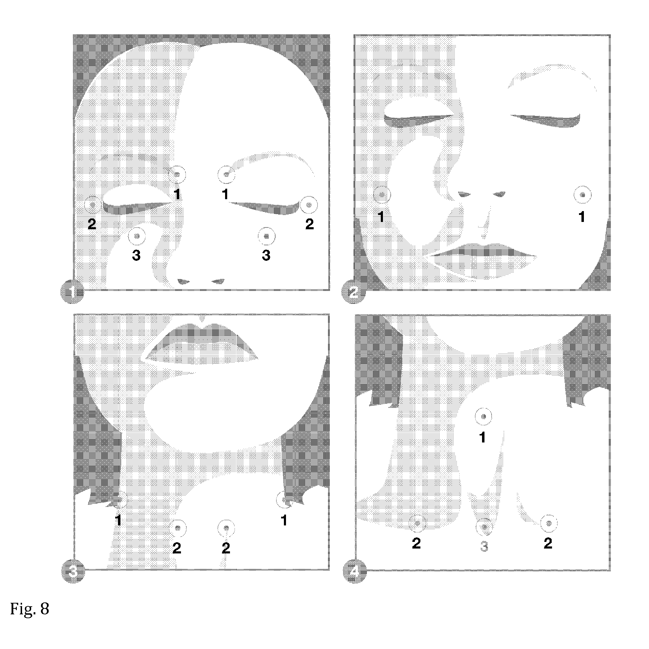

Fig. 8 zeigt die Lage der Akupressur-Punkte in den folgenden Atrophie-Regionen
Abb. 1: Magen-Region
   1. Blase 2: Am inneren Ende der Augenbrauen
   2. Gallenblase 1: 0,5 eigene Daumenbreiten seitlich der äußeren Augenwinkel
   3. Magen 2: Senkrecht unter den Pupillen, knapp oberhalb der Wangenknochen
Abb.2: Dünndarm-Region
   1. Dünndarm 18: Auf Höhe des unteren Nasenrandes und unterhalb der äußeren Augenwinkel
Abb. 3: Magen-Darm-Region
   1. Dünndarm 16: Hinterer Rand der seitlichen Halsmuskeln auf der Höhe der Spitze des Adamsapfels
   2. Magen 9: Auf Höhe der Spitze des Adamsapfels und am vorderen Rand der seitlichen Halsmuskeln
Abb. 4: Ren-Mai-Zone / Untere Magen-Region
   1. Ren Mai 23: Im Grübchen oberhalb des Adamsapfels
   2. Extra Punkt 4: Oberer Rand des Schlüsselbeins und am äußeren Rand des seitlichen Kopfnickers
   3. Ren Mai 22: In einem Grübchen, eine halbe Daumenbreite oberhalb des Brustbeins

## Patentansprüche

1. Pflaster zur Aufbringung auf die Haut für kosmetische Zwecke mit einer Klebeschicht, **dadurch gekennzeichnet, dass** auf der Klebeschicht ein oder mehrere Druckkörper mit einem mittleren Durchmesser von 0,5 bis 5 mm angeordnet sind.

2. Pflaster nach Anspruch 1, **dadurch gekennzeichnet, dass** die Klebeschicht eine nicht klebende Deckschicht aufweist.

3. Pflaster nach Anspruch 2, **dadurch gekennzeichnet, dass** zwischen Deckschicht und eine Klebeschicht eine Matrixschicht angeordnet ist und die Matrixschicht eine oder mehrere Druckkörper aufweist.

4. Pflaster nach Anspruch 1 oder 3, **dadurch gekennzeichnet, dass** die Druckkörper aus nicht-ferromagnetischen Metallen, Keramik, Polymeren, Holz oder Pflanzensamen bestehen.

5. Pflaster nach einem der Ansprüche 1 bis 4, **dadurch gekennzeichnet, dass** die Druckkörper weitgehend kugelförmig sind.

6. Pflaster nach einem der Ansprüche 1 bis 5, **dadurch gekennzeichnet, dass** die Pflaster eine Fläche von 0,5 bis 4 cm² aufweisen.

7. Pflaster nach einem der Ansprüche 3 bis 6, **dadurch gekennzeichnet, dass** die die Matrixschicht ganz oder zumindest im Bereich der Druckkörper transparent ist.

8. Pflaster nach einem der Ansprüche 1 bis 7, **dadurch gekennzeichnet, dass** die Deckschicht, die Klebeschicht und/oder die Matrixschicht aromatische Öle oder Parfüms enthalten.

9. Pflaster nach einem der Ansprüche 1 bis 8, **dadurch gekennzeichnet, dass** die Deckschicht, die Klebeschicht und die Matrixschicht durchlässig für Luft und Feuchtigkeit sind.

10. Pflaster nach einem der Ansprüche 1 bis 9, **dadurch gekennzeichnet, dass** die Klebeschicht wasserresistent ist.

11. Verfahren zur kosmetischen Akupressur der menschlichen Haut durch Aufkleben eines Pflasters nach den Ansprüchen 1 bis 10 auf die menschliche Haut für 1 bis 12 Stunden.

12. Verfahren nach Anspruch 11, **dadurch gekennzeichnet, dass** durch die kosmetische Akupressur eine kosmetische Behandlung von Hautfalten, Lifting von atrophierter Haut, eine kosmetische Behandlung von altersbedingter Fleckenbildung auf der Haut und/oder eine kosmetischen Behandlung von sichtbaren Äderchen durchgeführt wird.
